# EUROPEAN PATENT SPECIFICATION

(11) **EP 3 496 808 B1**
(45) Date of publication and mention of the grant of the patent: **10.06.2020**
(21) Application number: 17755006.8
(22) Date of filing: 11.08.2017
(51) Int. Cl.: A61N 1/365, A61B 5/02

(54) **DIASTOLIC ENDOCARDIAL ACCELERATIONS FOR HEART FAILURE MONITORING**
DIASTOLISCHE ENDOKARDIALE BESCHLEUNIGUNGEN ZUR HERZFEHLERÜBERWACHUNG
ACCÉLÉRATIONS ENDOCARDIAQUES DIASTOLIQUES DESTINÉES À LA SURVEILLANCE DE L'INSUFFISANCE CARDIAQUE

(30) Priority: 11.08.2016 US 201662373461 P
(43) Date of publication of application: 19.06.2019
(73) Proprietor: Cardiac Pacemakers, Inc., St. Paul, MN 55112-5798 (US)
(72) Inventor: THAKUR, Pramodsingh Hirasingh, Woodbury, Minnesota 55129 (US); KOOP, Brendan Early, Ham Lake, Minnesota 55304 (US); AN, Qi, Blaine, Minnesota 55449 (US); MAILE, Keith R., New Brighton, MN 55112 (US)
(74) Representative: Pfenning, Meinig & Partner mbB
(86) International application number: PCT/US2017/046542
(87) International publication number: WO 2018/031906

(56) References cited:
- WO-A1-2014/064267
- US-B1- 6 810 284

## Description

### TECHNICAL FIELD

This document relates generally to medical devices, and more particularly, to systems and methods for monitoring patients with a heart failure.

### BACKGROUND

Congestive heart failure (CHF) is a leading cause of death in the United States. CHF occurs when the heart is unable to adequately supply enough blood to maintain a healthy physiological state. CHF may be treated by drug therapy, or by an implantable medical device (IMD) such as for providing cardiac pacing therapies, including resynchronization therapy (CRT) to correct cardiac dyssynchrony within a ventricle or between ventricles.

Some IMDs may be used to monitor CHF patients and detect events leading to worsening of heart failure. Frequency patient monitoring may help reduce heart failure hospitalization. Identification of patient at an elevated risk of developing future heart failure events such as heart failure decompensation may help ensure timely treatment, thereby improving prognosis and patient outcome. Identifying and safely managing the patients having risk of future heart failure events can avoid unnecessary medical intervention and reduce healthcare cost.

Some IMDs may chronically stimulate excitable tissues or organs, such as a heart, to help restore or improve cardiac performance in a patient with CHF, or to treat abnormal cardiac rhythms. Such ambulatory medical devices may have electrodes that may be positioned within the heart or on a surface of the heart. The electrodes may be electrically coupled to an electronics unit such as a pulse generator, such as via a lead, and may be used to deliver electrostimulation to the heart. WO2014064267 discloses a control system for a cardiac assist device including a motion sensor connected to the wall of the right ventricle and measurement of diastolic function based on the motion sensor signal.

### SUMMARY

The invention is defined in claim 1. An ambulatory medical device (AMD), such as an implantable medical device, a subcutaneous medical device, a wearable medical device, or other external medical device, may be used to monitor heart failure (HF) patient, detect worsening heart failure, or deliver a therapy to restore or improve the cardiac function. The AMD may include implanted leads such as transvenous leads that include electrodes for cardiac sensing or for therapy delivery. Some device malfunction may be related to degradation of lead integrity. In some patients, implantable leads such as transvenously implanted leads may cause infection or other complications.

The AMD may include physiological sensors that may sense electrical or mechanical activities of the heart, or other physiological variables indicative of cardiac function. Among the sensed physiological signals, heart sounds signals may provide information about systolic and diastolic function of the heart. Heart sounds are associated with mechanical vibrations from activity of a patient's heart and the flow of blood through the heart. Heart sounds recur with each cardiac cycle and are separated and classified according to the activity associated with the vibration. A heart sounds signal may include one or more components within a cardiac cycle, including a first (S1), a second (S2), a third (S3), or a fourth (S4) heart sound. The S1 is associated with the vibrational sound made by the heart during tensing of the mitral valve. The S2 marks the beginning of diastole. The S3 is related to filling pressures of the left ventricle during diastole. The S4 is associated with atria contraction such as to overcome an abnormally stiff ventricle.

Monitoring heart sounds, such as S3, may be useful in assessing HF status. Heart failure patients may have fluid accumulation in the lungs that may cause an elevation of ventricular filling pressure. When HF status worsens, the diastolic function of the heart deteriorates. This may be accompanied by a louder than normal S3. A heart sound-based system for cardiac monitoring and detection of worsening heart failure may require reliable and accurate detection of heart sound components, such as S3 for assessing diastolic function of the heart. The present inventors have recognized a need of systems and methods that may effectively monitor cardiac function and accurately detect worsening heart failure, while at the same time may avoid or mitigate potential adverse effects associated with implantable leads.

This document discusses, among other things, a patient management system for monitoring patients with cardiac diseases. The system may include an accelerometer sensor for sensing epicardial or endocardial acceleration (EA) signal. The accelerometer may be included in a leadless medical device (LMD) or a lead-based medical device or be positioned on a lead electrically coupled to the lead-based medical device. The system may additionally include a companion device in communication with the accelerometer sensor via a communication network such as a wireless communication channel. The companion device may sense a cardiac signal such as a heart sound signal, and use the cardiac signal and the EA signal transmitted from the leadless or lead-based medical device to generate an indicator of diastolic function of the heart. The system may provide the diastolic function indicator to a user or a process, or to detect worsening of heart failure based on the diastolic function indicator. Sensing the EA signal and assessment of cardiac diastolic function using the EA signal, as described herein, can be used to improve operation or programming of a medical device including or coupled to a sensor for sensing the EA signal.
Example 1 is a system that may comprise an accelerometer sensor configured to sense an epicardial or endocardial acceleration (EA) signal from a portion of a heart, the EA signal indicative of one or more heart sound components. The system may optionally include a processor circuit configured to generate a diastolic function indicator (DF) using the sensed EA signal. In other examples, the system may optionally include a first communication circuit communicatively coupled to a companion device via a wireless communication link. The first communication circuit may be configured to receive a sense command from the companion device for sensing the EA signal and to transmit the sensed EA signal to the companion device.
In Example 2, the subject matter of Example 1 optionally includes a leadless medical device, where the accelerometer sensor may be configured to attach to and substantially conform to a surface of the leadless medical device.
In Example 3, the subject matter of Example 2 optionally include the leadless medical device that may include an anchoring mechanism for positioning the leadless medical device on an endocardial or epicardial surface of one of a left ventricle, a right ventricle, a left atrium, or a right atrium.
In Example 4, the subject matter of any one or more of Examples 1-3 optionally include a therapy circuit configured to generate a therapy to the patient in response to a therapy command from the companion device.
In Example 5, the subject matter of Example 1 optionally includes the first communication circuit configured to receive the sense command that may include time to start and terminate sensing of the EA signal, or electrodes used for sensing the EA signal.
In Example 6, the subject matter of any one or more of Examples 1-5 optionally include the companion device that may include: a physiological sensor circuit configured to sense a cardiac signal from the heart; a second communication circuit configured to receive the sensed EA signal from the accelerometer sensor via the wireless communication link; and a processor circuit configured to generate a diastolic function indicator (DFI) using the cardiac signal and the sensed EA signal; and an output unit configured to provide the DFI to a user or a process.
In Example 7, the subject matter of Example 6 optionally includes the companion device that may include an implantable cardiac device.
In Example 8, the subject matter of any one or more of Examples 6-7 optionally include the processor circuit of the companion device that may be configured to detect worsening heart failure using the DFI.
In Example 9, the subject matter of any one or more of Examples 6-8 optionally include the physiological sensor circuit that may include a heart sound (HS) sensor to sense a HS signal; and the processor circuit is configured to generate the DFI using the sensed EA signal and the sensed HS signal.
In Example 10, the subject matter of any one or more of Examples 6-9 optionally include the processor circuit that may be configured to determine whether the EA signal is sensed during cardiac sensing or cardiac pacing, and to generate the DFI using at least a portion of the EA signal during the cardiac sensing.
In Example 11, the subject matter of Example 9 optionally include the physiological sensor circuit that may include: a cardiac activation sensor to sense cardiac activations; and the processor circuit is configured to generate the DFI using timings of cardiac activations and the HS signal.
In Example 12, the subject matter of Example 11 optionally includes the processor circuit of the mater device that may be configured to determine HS detection windows for one or more of first (S1), second (S2), third (S3), or fourth (S4) heart sounds using the timings of cardiac activations, and to detect the one or more heart sounds within the respective HS detection windows; and generate the DFI using the detected one or more heart sounds.
In Example 13, the subject matter of any one or more of Examples 1-12 optionally include a first leadless medical device configured to be positioned on an endocardial or epicardial surface of a left ventricle (LV) of the heart to sense a LV activation, and a second leadless medical device configured to be positioned on an endocardial or epicardial surface of a right ventricle (RV) of the heart to sense a RV activation. The processor circuit of the companion device may be configured to generate the DFI using a temporal relationship between the sensed LV activation and the sensed RV activation.
In Example 14, the subject matter of any one or more of Examples 1-13 optionally include a first leadless medical device configured to be positioned on an endocardial or epicardial surface of an atrium of the heart and a second leadless medical device configured to be positioned on an endocardial or epicardial surface of a ventricle of the heart. The first leadless medical device may include an accelerometer sensor to sense an atrial EA signal. The second leadless medical device may include an accelerometer sensor to sense a ventricular EA signal. The processor circuit of the companion device may be configured to generate the DFI using an intensity relationship between the sensed atrial EA signal and the sensed ventricular EA signal.
In Example 15, the subject matter of Example 14 optionally includes the DFI that may include a ratio of an intensity of the atrial EA signal to an intensity of the ventricular EA signal.
Example 16 is a method of operating a medical device positioned at an endocardial or epicardial surface of a heart. The method may comprise the steps of establishing a wireless communication link between the medical device and a companion device; sensing an epicardial or endocardial acceleration (EA) signal from the heart in response to a sense command from the companion device, the EA signal indicative of one or more heart sound components; transmitting the sensed EA signal to the companion device via the wireless communication link; and generating a diastolic function indicator (DFI) at the companion device using the sensed EA signal.
In Example 17, the subject matter of Example 16 optionally includes sensing a heart sound (HS) signal via the companion device. The DFI may be generated using both the EA signal and the HS signal.
In Example 18, the subject matter of Example 17 optionally includes sensing cardiac activations via the medical device in response to the sense command, and determining HS detection windows for detection of one or more of first (S1), second (S2), third (S3), or fourth(S4) heart sounds using timings of cardiac activations. The sensing of the HS signal via the companion device may include detecting the one or more heart sounds within the respective HS detection windows, and the DFI may be generated using the detected one or more heart sounds.
In Example 19, the subject matter of Example 16 optionally include: sensing a left ventricular (LV) activation via a first leadless medical device positioned on an endocardial or epicardial surface of the LV of the heart; sensing a right ventricular (RV) activation via a second leadless medical device positioned on an endocardial or epicardial surface of the RV of the heart; and determining a temporal relationship between the sensed LV activation and the sensed RV activation. The DFI may be generated using the temporal relationship.
In Example 20, the subject matter of Example 16 optionally include: sensing an atrial EA signal via a first leadless medical device configured to be positioned on an endocardial or epicardial surface of an atrium of the heart; sensing a ventricular EA signal via a second leadless medical device configured to be positioned on an endocardial or epicardial surface of a ventricle of the heart; and determining an intensity ratio of an intensity of the atrial EA signal to an intensity of the ventricular EA signal. The DFI may be generated using the intensity ratio.
In Example 21, the subject matter of Example 16 optionally include receiving a therapy command from the companion device via the wireless communication link, and generating and delivering a therapy via the medical device to the patient in response to the therapy command.

This Summary is an overview of some of the teachings of the present application and not intended to be an exclusive or exhaustive treatment of the present subject matter. Further details about the present subject matter are found in the detailed description and appended claims. Other aspects of the invention will be apparent to persons skilled in the art upon reading and understanding the following detailed description and viewing the drawings that form a part thereof, each of which are not to be taken in a limiting sense. The scope of the present invention is defined by the appended claims and their legal equivalents.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 illustrates generally an example of a patient management system and portions of an environment in which the patient management system may operate.
FIG. 2 illustrates generally an example of a cardiac function monitoring system for monitoring cardiac function such as a progression of a heart failure.
FIG. 3 illustrates generally an example of a networked system for monitoring cardiac function such as a progression of a heart failure.
FIG. 4 illustrates generally an example of a method for monitoring cardiac function such as a progression of a heart failure using an LMD and a companion device.
FIG. 5 illustrates generally an example of a method for monitoring cardiac synchronization.
FIG. 6 illustrates generally an example of a method for monitoring ventricular filling functionality including an echocardiographic metric of early to late ventricular filling velocity ratio.

### DETAILED DESCRIPTION

Disclosed herein are systems, devices, and methods for monitoring patients with cardiac diseases such as heart failure. The system may include an accelerometer sensor for sensing an epicardial or endocardial acceleration (EA) signal, and a companion device that senses a cardiac signal such as a heart sound signal. The accelerometer may be included in a leadless or a lead-based medical device. The leadless or lead-based medical device can be communicatively coupled to the companion device via a wireless communication link. The companion device may determine a diastolic function indicator using the cardiac signal and the EA signal, and detect worsening of heart failure based on the diastolic function indicator.

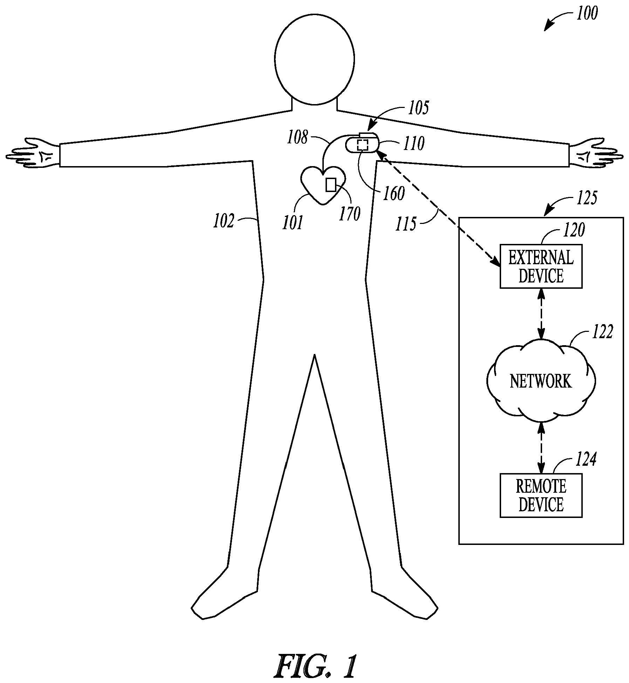

FIG. 1 illustrates generally an example of a patient management system 100 and portions of an environment in which the patient management system 100 may operate. The patient management system 100 may include an ambulatory system 105 associated with a patient body 102, an external system 125, and a telemetry link 115 providing for communication between the ambulatory system 105 and the external system 125.

The ambulatory system 105 may include an ambulatory medical device (AMD) 110 and a therapy delivery system such as a lead system 108. The AMD 110 may include an implantable device that may be implanted within the body 102 and coupled to a heart 101 via the lead system 108. Examples of the implantable device may include, but are not limited to, pacemakers, pacemaker/defibrillators, cardiac resynchronization therapy (CRT) devices, cardiac remodeling control therapy (RCT) devices, neuromodulators, drug delivery devices, biological therapy devices, diagnostic devices such as cardiac monitors or loop recorders, or patient monitors, among others. The AMD 110 may alternatively or additionally include subcutaneously implanted devices such as a subcutaneous ICD or a subcutaneous diagnostic device, wearable medical devices, or other external monitoring or therapeutic medical devices such as a bedside monitor.

The lead system 108 may include one or more transvenously, subcutaneously, or non-invasively placed leads or catheters. Each lead or catheter may include one or more electrodes. The arrangements and uses of the lead system 108 and the associated electrodes may be determined based on the patient need and the capability of the AMD 110. The lead system 108 and the associated electrodes may deliver therapy to treat cardiac or pulmonary diseases. The therapies may include pacing, cardioversion, defibrillation, neuromodulation, drug therapies, or biological therapies, among other types of therapies. In an example, the electrodes on the lead system 108 may be positioned inside or on a surface of at least a portion of the heart, such as a right atrium (RA), a right ventricle (RV), a left atrium (LA), a left ventricle (LV), or any tissue between or near the heart portions. In an example, the lead system 108 and the associated electrodes may be implanted subcutaneously or wearable on the patient body. The associated electrodes on the lead system 108 may be positioned at the patient's thorax or abdomen to sense intrinsic physiological signals indicative of cardiac or pulmonary activities, or physiological responses to diagnostic or therapeutic stimulations to a target tissue.

The AMD 110 may house an electronic circuit for sensing a physiological signal, such as by using a physiological sensor or the electrodes associated with the lead system 108. Examples of the physiological signal may include one or more of electrocardiogram, intracardiac electrogram, arrhythmia, heart rate, heart rate variability, intrathoracic impedance, intracardiac impedance, arterial pressure, pulmonary artery pressure, left atrial pressure, RV pressure, LV coronary pressure, coronary blood temperature, blood oxygen saturation, one or more heart sounds, intracardiac acceleration, physical activity or exertion level, physiological response to activity, posture, respiration rate, tidal volume, respiratory sounds, body weight, or body temperature. The AMD 110 may initiate or adjust therapies based on the sensed physiological signals.

The patient management system 100 may include a cardiac monitor 160 for monitoring cardiac function. The cardiac monitor 160 may analyze the diagnostic data such as acquired by the ambulatory system 105 for patient monitoring, risk stratification, and detection of events indicating presence, onset, termination, improvement, or worsening of a cardiac condition, such as heart failure. By way of non-limiting example and as illustrated in FIG.1, the cardiac monitor 160 may be substantially included in the AMD 110. Alternatively, the cardiac monitor 160 may be substantially included in the external system 125, or be distributed between the ambulatory system 105 and the external system 125.

The patient management system 100 may include a leadless medical device (LMD) 170. The LMD may include a device body, and one or more electrodes positioned on an outer surface of the device body. Unlike a tethered device where the sensing or stimulation electrodes are substantially away from, and connected via a lead wire to, a device body, the LMD requires no lead, wire, or tether extended between the electrodes and the device body. The LMD may include an anchoring or fixation mechanism for positioning the LMD on an endocardial surface of one of a left ventricle, a right ventricle, a left atrium, or a right atrium. Examples of the anchoring or fixation mechanism may include one or more pins, staples, threads, screws, helix, or tines, among other fixation structures. In some examples, the LMD may additionally or alternatively be configured to be securely positioned on an epicardial surface of a portion of the heart. In an example, the LMD may be delivered transvenously and positioned within a blood vessel on the heart, such as a coronary vein, where one or more electrodes on the LMD may be directly or indirectly in contact with the epicardial surface of the heart.

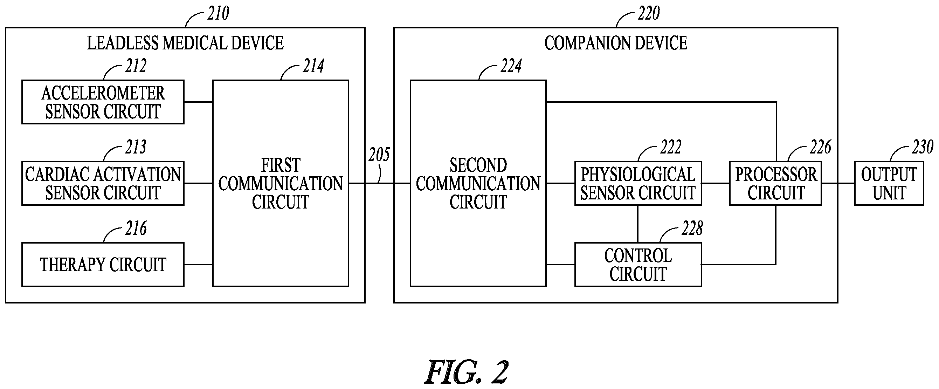

The device body of the LMD 170 may house circuitry that couples to the one or more electrodes to sense cardiac electrical activity. The LMD 170 may additionally include one or more physiological sensors configured to sense one or more physiological signals. Examples of the sensors may include an accelerometer, a blood pressure sensor, a heart sound sensor, a blood-oxygen sensor, a temperature sensor, a flow sensor, a pressure sensor, a chemical sensor, or any other suitable sensors that are configurable to measure one or more physiological parameters of the patient. In an example, the LMD 170 may include an accelerometer sensor configured to sense an epicardial or endocardial acceleration (EA) signal from a portion of a heart. The EA signal, or the peak endocardial acceleration (PEA) signal, may be indicative of force generated while the heart (or a portion such as right or left ventricle) contracts or relaxes, and thus may provide information of cardiac systolic or diastolic function. The EA signal may be correlated to one or more heart sound components, such as the S1, S2, S3 or S4 heart sounds. The LMD 170 may include circuitry that enables communication between the LMD 170 and a companion device, such as the AMD 110. Through an established communication link, the LMD 170 may perform various functionalities including, for example, receive a sense command from the AMD 110 for sensing the EA signal, or transmit the sensed EA signal to the AMD 110. Examples of the LMD 170 and communication with the companion device are discussed below, such as with reference to FIG. 2.

The LMD 170 may further include a therapy circuit configured to generate a therapy to the patient, such as electrical stimulation therapy for stimulating a target cardiac tissue, neural tissue, or other tissues that the electrodes of the LMD are in contact with or have an effect on. In an example, the LMD 170 may deliver cardiac pacing therapy from a region inside a heart chamber or on the epicardial surface of the heart. An example of such an LMD may include the leadless cardiac pacemaker (LCP) disclosed in the commonly assigned U.S. Patent Application Publication US2016/0051823 by Maile et al., entitled "LEADLESS CARDIAC PACEMAKER HAVING A SENSOR WITH A LOWER POWER MODE," which is hereby incorporated by reference in its entirety.

The external system 125 may be used to program the AMD 110. The external system 125 may include a programmer, or a patient management system that may access the ambulatory system 105 from a remote location and monitor patient status and/or adjust therapies. By way of non-limiting example, the external system 125 may include an external device 120 in proximity of the AMD 110, a remote device 124 in a location relatively distant from the AMD 110, and a telecommunication network 122 linking the external device 120 and the remote device 124. The telemetry link 115 may be an inductive telemetry link, a capacitive telemetry link, or a radio-frequency (RF) telemetry link. The telemetry link 115 may provide for data transmission from the AMD 110 to the external system 125. This may include, for example, transmitting real-time physiological data acquired by the AMD 110, extracting physiological data acquired by and stored in the AMD 110, extracting patient history data such as data indicative of occurrences of arrhythmias, occurrences of decompensation, and therapy deliveries recorded in the AMD 110, and extracting data indicating an operational status of the AMD 110 (e.g., battery status and lead impedance). The telemetry link 115 may also provide for data transmission from the external system 125 to the AMD 110. This may include, for example, programming the AMD 110 to perform one or more functions including acquiring physiological data, performing self-diagnostic test (such as for a device operational status), establishing communications with the LMD 170, analyzing the physiological data, or delivering a therapy, among others. In some examples, the external system 125 may be used to program the LMD 170, such as to program cardiac activity sensing or therapy delivery via the one or more electrodes, or sensing a physiological signal via a physiological sensor included in the LMD 170. The patient management system 100 may optionally include another external system (not shown) separate from the external system 125, which is dedicated for programming the LMD 170.

Although the discussion herein with respect to the patient management system 100 focuses on leadless medical device such as the LMD 170, this is meant only by way of example and not limitation. It is within the contemplation of the inventors, and within the scope of this document, that a lead-based medical device may be used. In an example, an accelerometer may be included in the lead-based medical device or a positioned on a lead electrically coupled to the lead-based medical device to sense an EA signal from a portion of a heart. The lead-based medical device may receive a sense command from the AMD 110 for sensing the EA signal, transmit the sensed EA signal to the AMD 110, or generate and deliver a therapy to the patient.

Portions of the AMD 110 or the external system 125 may be implemented using hardware, software, or any combination of hardware and software. Portions of the AMD 110 or the external system 125 may be implemented using an application-specific circuit that may be constructed or configured to perform one or more particular functions, or may be implemented using a general-purpose circuit that may be programmed or otherwise configured to perform one or more particular functions. Such a general-purpose circuit may include a microprocessor or a portion thereof, a microcontroller or a portion thereof, or a programmable logic circuit, or a portion thereof. For example, a "comparator" may include, among other things, an electronic circuit comparator that may be constructed to perform the specific function of a comparison between two signals or the comparator may be implemented as a portion of a general-purpose circuit that may be driven by a code instructing a portion of the general-purpose circuit to perform a comparison between the two signals.

FIG. 2 illustrates generally an example of a cardiac function monitoring system 200 for monitoring cardiac function, such as a progression of a heart failure or other cardiac disease. The cardiac monitoring system 200 may provide diagnostic decisions, recommend treatment, or deliver therapies based on the cardiac monitoring. The cardiac monitoring system 200 may be distributedly implemented between the AMD 110 and LMD 170, or among the AMD 110, LMD170, and the external system 125. The cardiac monitoring system 200 may include one or more of a leadless medical device (LMD) 210, a companion device 220, and an output unit 230.

The LMD 210, which may be an embodiment of the LMD 170, may include an accelerometer sensor circuit 212, a cardiac activation sensor circuit 213, and a first communication circuit 214. The accelerometer sensor circuit 212 may be coupled to an accelerometer sensor, such as a two-axis or a three-axis accelerometer sensor. Examples of the accelerometer may include flexible piezoelectric crystal (e.g., quartz) accelerometer or capacitive accelerometer, fabricated using micro electro-mechanical systems (MEMS) technology. The accelerometer sensor may be encapsulated within the device body of the LMD 170. In an example, the accelerometer may be sized, shaped, or otherwise configured to attach to and substantially conform to a surface of the LMD 170, such as at least partially wrapping along a contour of the device body of the LMD 170.

The accelerometer sensor circuit 212 may be configured to sense an epicardial or endocardial acceleration (EA) signal from a portion of a heart, such as on an endocardial or epicardial surface of one of a left ventricle, a right ventricle, a left atrium, or a right atrium. The accelerometer sensor circuit 212 may include one or more sub-circuits to amplify, digitize, filter, or perform other signal conditioning operations on the sensed EA signal. The EA signal may be indicative of one or more heart sound components that may be detected from a phonocardiogram. For example, within a cardiac cycle, the EA signal may include one or more of first (EA1), second (EA2), third (EA3), or fourth (EA4) acceleration component, corresponding to the S1, S2, S3, and S4 heart sounds, respectively. The EA1 may reflect the force generated following closure of the atrioventricular valves and opening of the semilunar valves, and the ventricular contractions. The EA2 corresponds to the force generated by the closure of the semilunar valves and is associated with the end of the ventricular systole. The EA3 may reflect vibrational forces of the ventricular walls during rapid filling. The EA4 may be related to the atrial contraction in during ventricular diastole. Like the S3 and S4 heart sounds, presence and accentuation of EA3 or EA4 components may be an indication of heart failure.

The cardiac activation sensor circuit 213 may be coupled to the one or more electrodes associated with the LMD 170 to sense cardiac activation, such as electrical depolarization of at least a portion of the heart. In an example, the cardiac activation sensor circuit 213 may sense an intrinsic cardiac electrogram and generate temporal or morphological features associated with a P wave, Q wave, R wave, QRS complex, or T wave. In an example, the cardiac activation sensor circuit 213 may sense an evoked cardiac electrogram, such as during cardiac stimulation, and generate temporal and intensity of evoked cardiac activations.

The first communication circuit 214 may be communicatively coupled to the companion device 220 via a wireless communication link 205. Examples of the wireless communication link 205 may include radiofrequency signals, inductive coupling, capacitive coupling, optical signals, acoustic signals, conducted communication signals, or any other signals suitable for communication. The first communication circuit 214 may communicate with the companion device 220, through the second communication circuit 224 included in the companion device 220, to accomplish one or more desired functions. For example, the first communication circuit 214 may communicate information such as sensed signals, data, messages, or instructions to the companion device 220, or receive from the companion device 220 a sense command to initiate a session of sensing the EA signal. The sense command may include time to start and to terminate the EA signal sensing, electrode selection or sensing vector configuration, sampling rate or sampling interval, among others instructions for signal sensing. The first communication circuit 214 may transmit the sensed EA signal to the companion device 220 via a wireless communication link 205, such as upon receiving a request command from the companion device 220. In some examples, the first communication circuit 214 may communicate information, such as sensed signals, data, messages, or instructions, with external system 125 or another external system separated from the external system 125 via the wireless communication link 205.

The LMD 210 may optionally include a therapy circuit 216 that may generate and deliver a therapy to the patient. The therapy may include electrostimulation therapy delivered a cardiac tissue, a nerve tissue, or other target tissues that the electrodes of the LMD are in contact with or have an effect on, or drug therapy including delivering drug to a tissue or organ. In an example, the electrostimulation therapy may include cardiac pacing therapy from a region inside a heart chamber or on the epicardial surface of the heart. The therapy circuit 216 may generate and deliver the therapy in response to a therapy command transmitted from the companion device 220 via the wireless communication link 205.

As previously discussed with respect to FIG. 1, the discussion of a leadless medical device such as the LMD 210 is meant only by way of example and not limitation. In some examples, a lead-based medical device may alternatively be used to replace the LMD 210 in cardiac monitoring system 200.

The companion device 220 may include an ambulatory medical device such as an implantable, a wearable, or a portable medical device, such as the AMD 110 in FIG. 1. The companion device 220 may include a physiological sensor circuit 222, a second communication circuit 224, a processor circuit 226, and a control circuit 228. Through the second communication circuit 224, the companion device 220 may receive the communicated signals, data, or messages from the LMD 210 via the wireless communication link 205, and perform various functions such as detecting progression of cardiac condition, generating medical diagnostic decisions, delivering therapies, storing or outputting the received data or processed information, or performing any other suitable functions.

The physiological sensor circuit 222 may include a sense amplifier to sense a physiological signal. The signal sensing may be performed when the heart undergoes an intrinsic rhythm such as a sinus rhythm, or when the heart undergoes stimulation. The physiological signal may include cardiac electrical signals such as electrocardiograms (ECGs) such as sensed by using electrodes non-invasively attached to the body surface, subcutaneous ECGs such as sensed by using subcutaneously placed electrodes, or intracardiac electrograms (EGMs) such as sensed by using electrodes on one or more leads such as the lead system 108 or the can housing of the AMD 110. The physiological signals may include signals indicative of cardiac mechanical activities such as contractions of an atrium or a ventricle as a response to an intrinsic heart rhythm or a stimulation of the heart. Examples of the signals indicative of cardiac mechanical activities may include heart sound signal or cardiac or thoracic impedance signal change as a result of cyclic cardiac contractions. In an example, the physiological sensor circuit 222 may be coupled to a respiratory sensor to sense a respiration signal and detect one or more respiration parameters such as one or more of a tidal volume, a respiration rate, a minute ventilation, a respiratory sound, or a rapid-shallow breathing index (RSBI) computed as a ratio of a respiratory rate measurement to a tidal volume measurement. The physiological sensor circuit 222 may additionally or alternatively sense hemodynamic signals via sensors that directly or indirectly measures dynamics of the blood flow in the heart chambers or in the blood vessels. Examples of the hemodynamic signals include arterial pressure, pulmonary artery pressure, left atrial pressure, RV pressure, LV coronary pressure; thoracic impedance or cardiac impedance; blood temperature; blood oxygen saturation; central venous pH value or oxygen or carbon dioxide level in the blood or other tissues or organs in the body, among others.

The physiological sensor circuit 222 may be coupled to one or more physiological sensors to sense a physiological signal. In an example, the physiological sensor circuit 222 may be coupled to a heart sound sensor to sense a heart sound signal including one or more of HS components such as first (S1), second (S2), third (S3), or fourth (S4) heart sound. The heart sound sensor may take the form of an accelerometer, an acoustic sensor, a microphone, a piezo-based sensor, or other vibrational or acoustic sensors. The HS sensor may be included in at least one part of an ambulatory system, such as the AMD 110, or a lead coupled to the ambulatory medical device such as the lead system 108. The physiological sensor circuit 222 may pre-process a sensed HS signal, including amplification, digitization, filtering, or other signal conditioning operations. In an example, the physiological sensor circuit 222 may include a bandpass filter adapted to filter the received HS signal to a frequency range of approximately between 5 and 90 Hz, or approximately between 9 and 90 Hz. In an example, the physiological sensor circuit 222 may include a double or higher-order differentiator configured to calculate a double or higher-order differentiation of the received HS signal.

The physiological sensor circuit 222 may compute an ensemble average of a HS signal over multiple physiological cycles such as multiple cardiac cycles, or over a specified time period such as one minute, ten minutes, one hour, one day, etc. The physiological sensor circuit 222 may generate respective time windows for detecting one or more HS components from an ensemble averaged HS signal. The time windows may be determined with reference to a physiological event such as Q wave, R wave, or QRS complexes detected from a surface ECG, a subcutaneous ECG, or cardiac sensing events in an intracardiac EGM.

As illustrated in FIG. 2, the physiological sensor circuit 222 may be coupled to the second communication circuit 224, and use at least some information received from the LMD 210 in sensing the physiological signals. In an example, the physiological sensor circuit 222 may receive cardiac activation, such as timing of the Q wave, R wave, QRS complex, or localized cardiac depolarization generate by the cardiac activation sensor circuit 213 of the LMD 210. The physiological sensor circuit 222 may determine the respective time windows for detecting HS components based on the timing information of the cardiac activations. By way of non-limiting example, an S1 detection window may begin at 50 milliseconds (msec) following an R wave (or a localized ventricular depolarization) and have a duration of 300 msec. An S2 detection window may begin at specified offset following a detected R wave (or a localized ventricular depolarization) or S1 heart sound. An S3 detection window may be determined using at least one cardiac signal feature such as the R-wave timing or the timing of S2 heart sound. The S3 detection window may have a specified duration and may begin at a specified offset following the detected S2. In an example, the offset may be 125 msec, and the S3 window duration may be 125 msec. The offset or the S3 window duration may be a function of a physiological variable such as a heart rate. For example, the offset may be inversely proportional to the heart rate, such that the S3 detection window may start at a smaller offset following the S2 at a higher heart rate.

In an example, the LMD 210 may be positioned at endocardial surface of a ventricle such as a right ventricle or a left ventricle of the heart. At least due to its close contact with the myocardial tissue at the ventricle, the sensing electrodes on the LMD 210 may be sensitive to cardiac depolarization. Timing of the cardiac activation sensed by the LMD 210 may therefore be more accurate than timing as determined from other cardiac signals such as subcutaneous ECG or surface ECG. The physiological sensor circuit 222 of the companion device 220 may detect the HS signal synchronously with the cardiac activation sensing by the cardiac activation sensor circuit 213 of the LMD 210. The physiological sensor circuit 222 may detect the HS components using respective time windows based on the timing information of ventricular activation such as provided by the LMD 210.

The physiological sensor circuit 222 may detect an HS component (such as S1, S2, S3 or S4 heart sound) from at least a portion of the HS signal within the respective HS detection window. For example, HS signal energy within a S3 detection window may be computed and compared to a S3 energy threshold, and an S3 component is detected in response to the HS signal energy exceeds the S3 energy threshold. In an example, the physiological sensor circuit 222 may detect an HS component adaptively by tracking the temporal locations of the previously detected HS features. For example, an S3 heart sound may be detected by adaptively tracking the timing of historically detected S3 heart sounds. A dynamic programming algorithm may be used to detect and track the S3 heart sound within the S3 detection window, such as that disclosed in the commonly assigned Patangay et al. U.S. Pat. No. 7,853,327 entitled "HEART SOUND TRACKING SYSTEM AND METHOD," which is hereby incorporated by reference in its entirety.

The processor circuit 226 may process the sensed physiological signals to generate a diastolic function indicator (DFI) indicating the diastolic function of the heart. In an example, the diastolic function indicator can include an indication of a stiffness of a chamber of the heart, such as a left ventricle (LV), a right ventricle (RV), a left atrium (LA), a right atrium (RA), or a component or portion of one or more of the atria or ventricles. In other examples, the DFI can include a measure of contractility of at least a portion of one or more of the atria or ventricles. In certain examples, the DFI can include a measurement of contractility, stiffness, or one or more other filling characteristic of the heart during filling portion of a cardiac cycle, absolute measurement or relative to one or more other measurement, such as during diastolic filling, or during a period corresponding to a third heart sound (S3). In other examples, the DFI can include a measurement of contractility, stiffness, or one or more other filling characteristic of the heart during one or more other portions of the cardiac cycle. The processor circuit 226 may be implemented as a part of a microprocessor circuit, which may be a dedicated processor such as a digital signal processor, application specific integrated circuit (ASIC), microprocessor, or other type of processor for processing information including physical activity information. Alternatively, the microprocessor circuit may be a general purpose processor that may receive and execute a set of instructions of performing the functions, methods, or techniques described herein.

The processor circuit 226 may include circuit sets comprising one or more other circuits or sub-circuits, that may, alone or in combination, perform the functions, methods, or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

The processor circuit 226 may be coupled to the physiological sensor circuit 222 to generate the DFI using the HS components generated from the physiological sensor circuit 222. In an example, the DFI may be generated using at least the S3 heart sound, such as S3 intensity (e.g., an amplitude of S3, or a peak of S3 signal power), or a trend of S3 intensity over a specified period of time. A louder S3, such as indicated by S3 intensity exceeding a threshold or an increasing trend of S3 intensity with an increase rate exceeding a threshold, may indicate a deterioration of diastolic function. The processor circuit 226 may detect worsening heart failure using the DFI, such as when the S3 intensity or the S3 trend satisfies a respective condition.

The processor circuit 226 may alternatively generate the DFI using the EA signal generated from accelerometer sensor circuit 212 of the LMD 210, including one or more EA components such as EA1, EA2, EA3, or EA4. As previously discussed, EA3 represents vibrational forces of the ventricular walls during rapid filling and corresponds to S3 heart sound. A louder EA3, such as indicated by the EA3 intensity exceeding a threshold or a rate of increase in EA3 intensity exceeding a rate threshold, may indicate a deteriorated diastolic function. The processor circuit 226 may detect worsening heart failure using the DFI, such as when the EA3 intensity or EA3 trend satisfies a respective condition.

As illustrated in FIG. 2, the processor circuit 226 may be coupled to both the physiological sensor circuit 222 and the second communication circuit 224, and generate the DFI using both the heart sounds and the EA signal or various EA components received from the LMD 210, referred to hereinafter as a collaborative DFI. The collaborative DFI may be computed using a combination of the EA3 intensity and the S3 intensity, or a combination of the EA3 intensity trend and the S3 intensity trend. Examples of the combination may be a linear or nonlinear combination, or other data fusion methods as generally known in the art.

In an example, the processor circuit 226 may determine whether the EA signal is sensed while the heart is stimulated (i.e., in a cardiac pacing session) or while the heart is not stimulated (i.e., in a cardiac sensing session). The determination may be based on the information generated by the therapy circuit 216 and the cardiac activation sensor circuit 213. If the LMD 210, via the therapy circuit 216, delivers cardiac pacing to the heart and the cardiac activation sensor circuit 213 detects a corresponding evoked electrical response, the EA signal sensed during cardiac pacing may be identified as a "paced EA" signal. If no cardiac pacing is delivered or no evoked electrical response is detected, the EA signal is sensed during cardiac sensing, and may be identified as a "sensed EA" signal. Information about the characterization of the EA signal (as either sensed EA or paced EA signal) may be transmitted to the companion device 220 via the wireless communication link 205. The processor circuit 226 may determine the corroborative DFI using the characterization of the EA signal. In an example, the processor circuit 226 may generate the DFI using only the sensed EA, without using the paced EA signal. The paced EA signal, as sensed during cardiac stimulation, may be susceptible to interferences and include undesirable pacing artifacts. Measurements taken from the paced EA signal, such as EA3 intensity or trends of EA3, may not adequately represent vibrational forces of the ventricular walls during rapid filling with sufficient fidelity. The processor circuit 226 may instead use heart sounds signal sensed at the physiological sensor circuit 222 when the heart is stimulated. The processor circuit 226 may generate the corroborative DFI using the EA3 intensity (or a trend thereof) from the sensed EA signal in the absence of cardiac stimulation, and switch to the S3 intensity (or a trend thereof) to generate the corroborative DFI when the cardiac pacing is delivered.

The control circuit 228 may control the operations of the physiological sensor circuit 222 and the processor circuit 226, and the data and instruction flow between these components. The control circuit 228 may also control the communication between the LMD 210 and the companion device 220, such as data and instruction (e.g., sensing or therapy instructions) sent to the LMD 210, or the data (e.g., EA signal and EA components, cardiac activation, or indication of therapy being delivered) received from the LMD 210. In an example, as an alternative of distinguishing the sensed EA from the paced EA at the processor circuit 226, the control circuit 228 may generate a request command to only receive the sensed EA signal or one or more EA components extracted therefrom, and not to receive the paced EA signal from the LMD 210.

The output unit 230 may provide the DFI to a user or a process. In an example, at least a portion of the output unit 230 may be implemented in the external system 125. The output unit 230 may generate a human-perceptible presentation of DFI, optionally along with other information such as S3 signal, EA signal, or the detection of worsening of heart failure (WHF). The output unit 230 may include a display for displaying the information. The information may be presented in a table, a chart, a diagram, or any other types of textual, tabular, or graphical presentation formats, for displaying to a system user. The presentation of the output information may include audio or other media format to alert the system user of the detected physiological events. In some examples, the companion device 220 may determine a therapy parameter, or adjust an existing therapy parameter, based on the DFI or the detected WHF. Examples of the therapy parameter may include electrodes or stimulation vector for use in delivering electrostimulation, pulse amplitude, pulse width, stimulation frequency, stimulation duration, or duty cycle, among others. The companion device 220 may program the LMD 210 to deliver a therapy according to the determined therapy parameter.

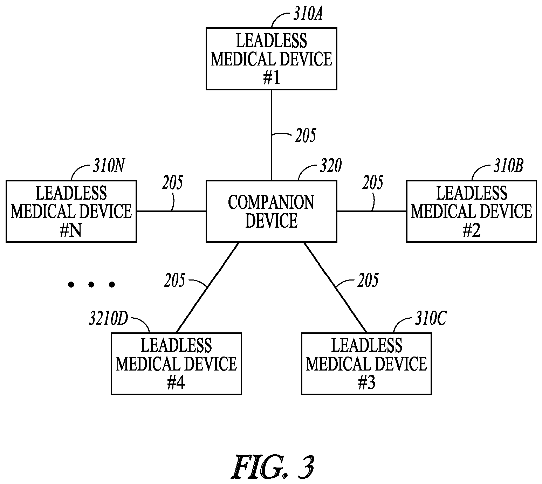

FIG. 3 illustrates generally an example of a networked system 300 for monitoring cardiac function, such as a progression of a heart failure or other cardiac disease. The networked system 300 may include a companion device 320 and a plurality of leadless medical devices (LMDs) such as 310A-310N. The companion device 320, which may be an embodiment of the companion device of 220 in FIG.2, may be in communication with the LMDs via the wireless communication link 205. The LMDs 310A-310N may be positioned at respective locations on endocardial or epicardial surfaces of the heart. In an example, some of the LMDs may be positioned at different heart chambers such as left atrium, left ventricle, right atrium, or right ventricle. In an example, some of the LMDs may be positioned at different epicardial or endocardial locations of the same heart chamber.

The LMDs 310A-310N may each be an embodiment of the LMD 210, which includes respective accelerometer sensor circuits to sense EA signal and one or more EA components. The companion device 320 may receive EA signal or EA components respectively from some or all of the LMDs 310A-N, and generate a diastolic function indicator (DFI) using the EA signals from the LMDs. The DFI may be determined using a combination of the EA3 components generated by at least some of the LMDs. In an example, the companion device 320 may generate a collaborative DFI using the EA3 components from at least some of the LMDs along with the S3 heart sound sensed by the physiological sensor circuit 222.

In an example, the companion device 320 may generate a collaborative DFI further using information about cardiac synchrony. The cardiac synchrony indicates temporal relationship of the cardiac activations in different parts of the heart. The cardiac synchrony may include atrioventricular (AV) synchrony between an atrium and a ventricle, inter-ventricular synchrony between left ventricle and right ventricle, or intraventricular synchrony between two locations on the same ventricle such as a left ventricle (LV) or a right ventricle (RV). Dyssynchrony, or compromised cardiac synchrony, may present in patients with heart failure. In an example, the first LMD 310A may be positioned on an endocardial or epicardial surface of the LV of the heart to sense a LV activation, and the second LMD 310B may be positioned on an endocardial or epicardial surface of the RV of the heart to sense a RV activation. The companion device 320 may receive timings of the LV activation and RV activation respectively from the LMDs 310A and 310B via the communication link 205, and determine a timing relation between the LV activation and the RV activation, such as a LV-RV delay within the same cardiac cycle. The companion device 320 may generate the DFI, or detect worsening heart failure, using the LV-RV delay, in lieu of or in addition to the heart sounds or EA signals.

In an example, the first LMD 310A may be positioned on an endocardial or epicardial surface of an atrium of the heart, and the second LMD 310B may be positioned on an endocardial or epicardial surface of a ventricle of the heart. The LMD 310A may include an accelerometer sensor to sense an atrial EA (aEA) signal. The LMD 310B may include an accelerometer sensor to sense a ventricular EA signal. The companion device 320 may receive atrial EA and ventricular EA (vEA) signals respectively from the LMDs 310A and 310B via the communication link 205, and determine an intensity relationship between the sensed aEA and vEA signals. In an example, the intensity relationship may be characterized by a ratio of an intensity of EA3 component of the aEA signal (denoted by aEA3) to an intensity of EA3 component of the vEA signal (denoted by vEA3). The aEA3 component indicates forces generated at the atrium during diastole, and vEA3 component indicates forces generated at the ventricle during diastole. The intensity ratio, aEA3/vEA3, may be correlated to the early to late ventricular filling velocity ratio (E/A ratio) which is ordinarily obtained from echocardiography. Abnormalities in the E/A ratio may suggest diastolic dysfunction where the left ventricle cannot fill with blood properly during the diastole period. The companion device 320 may generate the DFI, or detect worsening heart failure, using the intensity ratio, aEA3/vEA3, in lieu of or in addition to the EA3 signal intensity, EA3 trend, or the heart sounds.

In cardiac function monitoring system 200 in FIG. 2 or the networked system 300 in FIG. 3, the accelerometer sensor for sensing the EA signal is within the LMD 210 or the LMDs 310A-N. This is meant only by way of example and not limitation. In an example, the LMD 210, or at least some of the LMDs 310, may be positioned on a lead such as the lead system 108, and communicate with the companion device 220 or 320 via the lead in lieu of the wireless communication link 205. In an example, at least the accelerometer sensor may be positioned on a lead coupled to the companion device 220 or 320, such as the lead system 108 coupled to the AMD 110, to senses the EA signal. One or more of the accelerometer sensor circuit 212, the cardiac activation sensor circuit 213, or the therapy circuit 216 may be included in the companion device 220 or 320. The sensed EA signal may be transmitted to the companion device via the lead. The companion device 220 may generate the diastolic function indicator using the sensed EA signal, or additionally the cardiac signal sensed by the physiological sensor circuit 222, as previously discussed with reference to FIGS. 2 and 3.

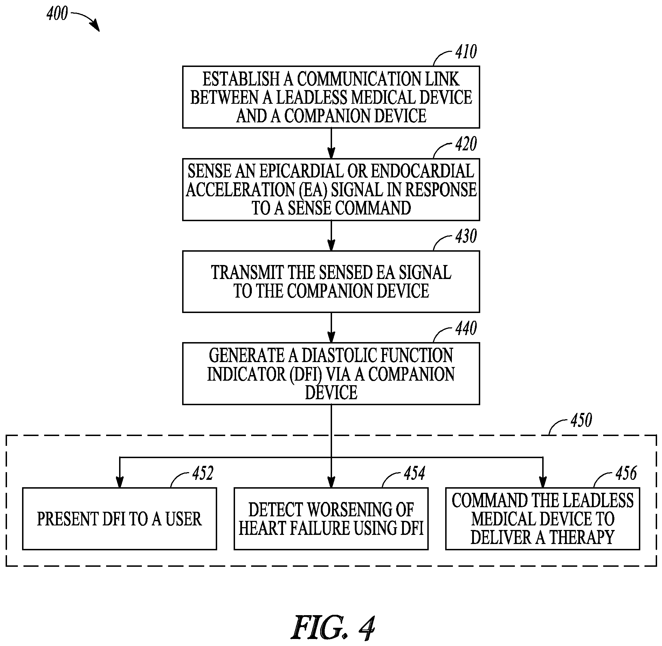

FIG. 4 illustrates generally an example of a method 400 for monitoring cardiac function, such as a progression of a heart failure or other cardiac disease. The method 400 may be implemented and operate in a system that comprises an ambulatory medical device (AMD) and a leadless medical device in wireless communication with the AMD, such as the cardiac function monitoring system 200 in FIG. 2, or a modification thereof.

The method 400 begins at 410 by establishing a communication link between a leadless medical device (LMD) and a companion device, such as the wireless communication link 205 between the LMD 210 and the companion device 220 as illustrated in FIGS. 2-3. The wireless communication may include radiofrequency (RF) signals, inductive coupling, capacitive coupling, optical signals, acoustic signals, conducted communication signals, or any other signals suitable for communication. At 420, an epicardial or endocardial acceleration (EA) signal may be sensed via the LMD. The EA signal may be indicative of peak force generated while the heart (or a particular portion such as right or left ventricle) contracts, and thus may provide useful information of cardiac function. The sensing of the EA signal may be intermittent, such as only during a period as specified in a sense command generated at the companion device and transmitted through the wireless communication link. The sense command may include time to start and to terminate the EA signal sensing, electrode selection and sensing vector configuration, sampling rate or sampling interval, among others instructions. Also at 420, one or more EA components may be extracted from the sensed EA signal, such as first (EA1), second (EA2), third (EA3), and fourth (EA4) components, which respectively correspond to S1, S2, S3, and S4 heart sounds.

At 430, the sensed EA signal, or one or more of EA components, may be transmitted to the companion device via the communication link. The transmission may be initiated in response to a request command from the companion device. The request may specify one or more EA components to transmit. In an example, EA3 may be requested and transmitted to the companion device for use to monitor heart failure progression. As previously discussed, EA3 may reflect vibrational forces of the ventricular walls during rapid filling and corresponds to S3 heart sound. A louder EA3 may indicate a deterioration of diastolic function, and thus may be used to detect worsening heart failure. In some examples, the request command may specify a particular EA signal such as sensed under particular conditions for selective transmission to the companion device. In an example, only the EA signal (or one or more EA components extracted therefrom) sensed when the heart is not stimulated, referred to as sensed EA signal, is selected to be transmitted to the companion device; the EA signals sensed when the heart is stimulated, referred to as paced EA signal, are not transmitted to the companion device. In some examples, the EA signals, regardless of pacing condition, may be transmitted to the companion device, along with information about characterization of the EA signal (as either sensed or paced EA), and the companion device may selected a portion of the EA signal such as only the sensed EA signal in absence of stimulation for use in cardiac function monitoring.

Information other than the EA signal may additionally be transmitted to the companion device, as specified in the request command. In an example, cardiac activation, such as electrical depolarization of at least a portion of the heart, may be sensed by the LMD, such as via the cardiac activation sensor circuit 213. Examples of cardiac activation may include intrinsic cardiac depolarization in a portion of the heart, or evoked response to cardiac stimulation. Information about timing and intensity of cardiac activation, such as a P wave, a Q wave, an R wave, a QRS complex, a T wave on an ECG or local cardiac depolarization may be transmitted to the companion device.

At 440, a diastolic function indicator (DFI) indicating cardiac diastolic function may be generated, such as via the companion device. The DFI may be determined using one or more of the EA signal sensed from the LMD, one or more physiological signals such as a HS signal sensed from the companion device, or a combination of at least an EA component and at least a HS component. In an example, the DFI may be computed using an intensity of the EA3 or a trend of EA3 intensity. An EA3 intensity exceeding a threshold or an increasing trend of EA3 intensity with a rate of increase exceeding a threshold may indicate deteriorated diastolic function. In another example, a HS signal may be sensed using a heart sound sensor at the companion device. The sensed HS signal may be amplified and filtered, and one or more HS components such as S1, S2, S3 or S4 heart sounds may be detected within respective time windows. In an example, the time windows may be determined with reference to a physiological event such as Q wave, R wave, or QRS complexes detected from a surface ECG, a subcutaneous ECG, or cardiac sensing events in an intracardiac EGM. In an example, the time windows may be determined using the timing information of the cardiac activations sensed by the LMD. The DFI may be computed using an intensity of S3 such as amplitude or a peak of S3 signal power, or a trend of S3 intensity over a specified period of time. A louder S3, such as indicated by S3 intensity exceeding a threshold or an increasing trend of S3 intensity with a rate of increase exceeding a threshold, may indicate a deterioration of diastolic function. In another example, the DFI may be computed using both the EA3 and the S3 heart sounds, such as a linear or nonlinear combination of the EA3 intensity and the S3 intensity, or a linear or nonlinear combination of the EA3 intensity trend and the S3 intensity trend.

The DFI may be used by several processes at 450. At 452, the DFI may be presented to a user, such as via the output unit 230 of FIG. 2. A human-perceptible presentation of DFI, optionally along with other information such as the sensed S3 signal, the sensed EA signal, or the detection of worsening of heart failure (WHF) may be displayed in a user-interface in various format including a table, a chart, a diagram, or any other types of textual, tabular, or graphical presentation formats. Additionally or alternatively, at 454 worsening heart failure (WHF) may be detected based on DFI. In an example, the WHF is detected if the EA3 intensity, a trend of EA3, S3 intensity, or a trend of S3 intensity exceeds a respective threshold or falls within a respective region, indicating a substantial deterioration of diastolic function. In another example, the WHF is detected if a corroborative DFI based on S3 and EA3 satisfies a specified condition. Additionally or alternatively, at 456 a therapy may be recommended or delivered to the patient based on the DFI or the detection of the worsening of HF. The therapy may include electrostimulation therapy delivered a cardiac tissue, a nerve tissue, or other target tissues that the electrodes of the LMD are in contact with or have an effect on, or drug therapy including delivering drug to a tissue or organ. In an example, the electrostimulation therapy may include cardiac pacing therapy from a region inside a heart chamber or on the epicardial surface of the heart. In an example, a therapy command may be generated from the companion device in response to the DFI satisfying a specified condition, which commands the LMD to deliver cardiac pacing at a specified time. In some examples, the companion device may determine a therapy parameter, or adjust an existing therapy parameter, based on the DFI or the detected WHF, and program the LMD to deliver a therapy according to the determined therapy parameter.

Although the discussion herein pertaining to method 400 focuses on one LMD, this is meant only by way of example and not limitation. It is within the contemplation of the inventors, and within the scope of the this document, that the method 400 may include method of using more than one LMD, such as a network of LMDs as illustrated in FIG. 3, to collaboratively monitor the diastolic function of the patient, including generating a DFI, detecting WHF, presenting the DFI to a user, recommending or delivering therapy to the patient, among other actions. In an example, one or more of the steps 410-430 of method 400 may similarly be implemented and executed respectively by two or more LMDs, and at 440 the DFI may be generated using the EA signals from two or more of the LMDs, or optionally together with the heart sounds, such as sensed by the companion device.

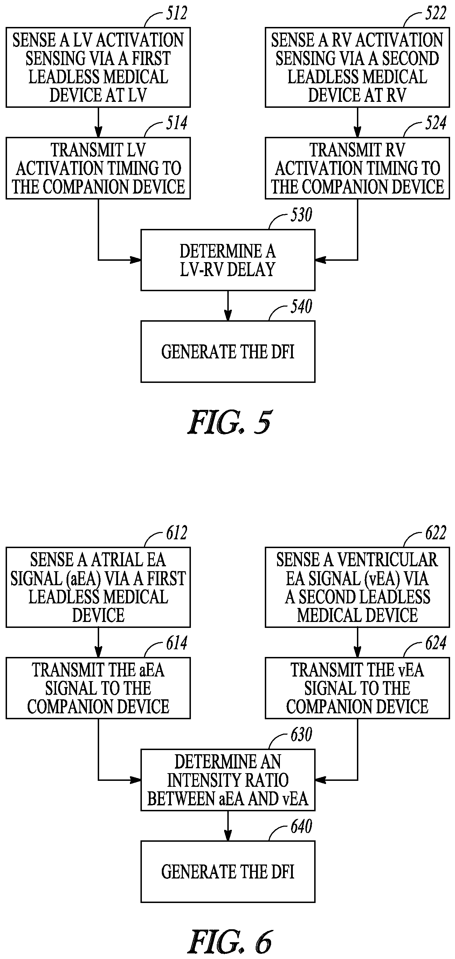

FIG. 5 illustrates generally an example of a method 500 for monitoring cardiac synchronization, such as an inter-ventricular synchronization between the left and right ventricles. The dyssynchrony, or compromised cardiac synchrony, may present in patients with heart failure. Detection of cardiac dyssynchrony may be useful in assessing worsening of heart failure. The method 500 may be used to generate a diastolic function indicator, and/or detect worsening of heart failure, in lieu of or in addition to the EA signals sensed by an LMD as discussed above in method 400 with reference to FIG. 4. In an example, the method 500 may be implemented and executed by the cardiac function monitoring system 200 as illustrated in FIG. 2, or a modification thereof.

At 512, a left ventricular (LV) activation may be sensed at an endocardial or epicardial surface of the LV of the heart, such as by using a first LMD 310A. The LV activation may be sensed using electrodes on the external surface of the LMD 310A, and the LV activation may include LV depolarization during an intrinsic rhythm (such as sinus rhythm) or when the heart undergoes electrical stimulation. At 514, information about the LV activation, including timing of the LV activation, may be transmitted to the companion device. Similarly, at 522, a right ventricular (RV) activation may be sensed at an endocardial or epicardial surface of the RV of the heart, such as by using a second LMD 310B. The RV activation may include LV depolarization sensed by using electrodes on the external surface of the LMD 310B when the heart undergoes intrinsic rhythm or an electrical stimulation. At 524, information about the RV activation, including timing of the RV activation, may be transmitted to the companion device.

At 530, a temporal relationship between the LV activation and the sensed RV activation, such as a LV-RV delay within the same cardiac cycle, may be determined. A DFI may be generated at 540 using the LV-RV delay. In an example, the DFI may be generated using a combination of the LV-RV delay and one or more of EA3 intensity or a trend of EA3 intensity, or S3 intensity or a trend of S3 intensity. The resulting DFI may then be used in forming a detection decision of worsening heart failure, or one or more processes such as illustrated at step 450 in FIG. 4.

FIG. 6 illustrates generally an example of a method 600 for monitoring ventricular filling functionality, such as an echocardiographic metric of early to late ventricular filling velocity ratio (E/A ratio). Abnormalities in the E/A ratio may suggest diastolic dysfunction where the left ventricle cannot fill with blood properly during the diastole period. Detection of ventricular filing function, such as a metric indicative or correlated to the E/A ratio, may be useful in assessing worsening of heart failure. The method 600 may be used to generate a quantity indicative of E/A ratio, and to detect worsening of heart failure in lieu of or in addition to the EA signals as discussed above in method 400, or the cardiac synchronization such as LV-RV delay as discussed above in method 500. In an example, the method 600 may be implemented and executed by the cardiac function monitoring system 200 as illustrated in FIG. 2, or a modification thereof.

At 612, an atrial EA signal may be sensed at an endocardial or epicardial surface of an atrium, such as a left or right atrium, such as by using an accelerometer sensor associated with a first LMD 310A. At 614, the atrial EA signal (aEA) or one or more components extracted from the aEA signal, may be transmitted to the companion device. Similarly, at 622, a ventricular EA signal may be sensed at an endocardial or epicardial surface of a left or right ventricle, such as by using an accelerometer sensor associated with a second LMD 310B. At 624, the ventricular EA signal (vEA) or one or more components extracted from the vEA signal, may be transmitted to the companion device.

At 630, an intensity relationship between the sensed aEA and vEA signals, or between a specified aEA component and a specified vEA component, may be determined. In an example, the intensity relationship may be characterized by a ratio of an intensity of the EA3 component of the aEA signal (denoted by aEA3) to an intensity of the E3 component of the vEA signal vEA intensity (denoted by vEA3). The aEA3 and vEA3 components respectively represents forces generated at the atrium and ventricular during diastole. The intensity ratio, aEA3/vEA3, may be correlated to the early to late ventricular filling velocity ratio (E/A ratio).

A DFI may be generated at 640, such as by using the intensity ratio, aEA3/vEA3. In an example, the DFI may be generated using a combination of the intensity ratio aEA3/vEA3 and one or more of EA3 intensity or a trend of EA3 intensity, or S3 intensity or a trend of S3 intensity as discussed above in method 400, or the cardiac synchronization such as LV-RV delay as discussed above in method 500. The resulting DFI may then be used in forming a detection decision of worsening heart failure, or one or more processes such as illustrated at step 450 in FIG. 4.

The above detailed description includes references to the accompanying drawings, which form a part of the detailed description. The drawings show, by way of illustration, specific embodiments in which the disclosure may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments may be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments may be combined with each other in various combinations or permutations. The scope of the disclosure should be determined with reference to the appended claims, along with the full scope of equivalents to which such claims are entitled.

## Claims

1. A system, comprising:
a leadless medical device including an accelerometer sensor configured to sense an epicardial or endocardial acceleration (EA) signal from a portion of a heart, the EA signal indicative of one or more heart sound components; and
a companion device separated from and communicatively coupled to the leadless medical device, the companion device including a processor circuit configured to generate a diastolic function indicator (DFI) using the sensed EA signal.

2. The system of claim 1, wherein the leadless medical device includes a first communication circuit communicatively coupled to the companion device via a wireless communication link, the first communication circuit configured to receive a sense command from the companion device for sensing the EA signal and to transmit the sensed EA signal to the companion device.

3. The system of any of claims 1-2, wherein the accelerometer sensor is configured to attach to and substantially conform to a surface of the leadless medical device,
wherein the leadless medical device includes an anchoring mechanism for positioning the leadless medical device on an endocardial or epicardial surface of one of a left ventricle, a right ventricle, a left atrium, or a right atrium.

4. The system of any of claims 1-3, wherein the leadless medical device includes a therapy circuit configured to generate a therapy to the patient in response to a therapy command from the companion device.

5. The system of claim2, wherein the sense command includes time to start sensing the EA signal and time to terminate sensing the EA signal, or electrodes used for sensing the EA signal.

6. The system of any of claims 1-5, wherein
the companion device includes:
a physiological sensor circuit configured to sense a cardiac signal from the heart;
a second communication circuit configured to receive the sensed EA signal from the accelerometer sensor via the wireless communication link; and
the processor circuit configured to generate the DFI using the cardiac signal and the sensed EA signal.

7. The system of claim 6, wherein:
the physiological sensor is configured to sense a HS signal; and
the processor circuit is configured to generate the DFI using the sensed EA signal and the sensed HS signal.

8. The system of claim7, wherein:
the physiological sensor circuit is further configured to sense cardiacactivation; and
the processor circuit is configured to generate the DFI using timing information of the sensed cardiac activation and the sensed HS signal.

9. The system of claim8, wherein the processor circuit is configured to:
determine HS detection windows for one or more of first (S1), second (S2), third (S3), or fourth (S4) heart sounds using the timings of cardiac activations;
detect the one or more heart sounds within the respective HS detection windows; and
generate the DFI using the detected one or more heart sounds.

10. The system of any of claims 1-9, comprising:
a first leadless medical device configured to be positioned on an endocardial or epicardial surface of a left ventricle (LV) of the heart to sense a LV activation; and
a second leadless medical device configured to be positioned on an endocardial or epicardial surface of a right ventricle (RV) of the heart to sense a RV activation,
wherein the processor circuit of the companion device is configured to generate the DFI using a temporal relationship between the sensed LV activation and the sensed RV activation.

11. The system of any of claims 1-9, comprising:
a first leadless medical device configured to be positioned on an endocardial or epicardial surface of an atrium of the heart, the first leadless medical device including an accelerometer sensor to sense an atrial EA signal; and
a second leadless medical device configured to be positioned on an endocardial or epicardial surface of a ventricle of the heart, the second leadless medical device including an accelerometer sensor to sense a ventricular EA signal; and
wherein the processor circuit of the companion device is configured to generate the DFI using an intensity relationship between the sensed atrial EA signal and the sensed ventricular EA signal.

12. The system of claim 11, wherein the DFI includes a ratio of an intensity of the atrial EA signal to an intensity of the ventricular EA signal.

13. The system of any of claims 1-12, wherein the companion device includes an implantable cardiac device.

14. The system of any of claims 1-13, wherein the processor circuit is configured to detect worsening heart failure using the DFI.

15. The system of any of claims 1-14, wherein the processor circuit is configured to:
determine whether the EA signal is sensed during cardiac sensing or cardiac pacing; and
generate the DFI using at least a portion of the EA signal only during the cardiac sensing.

## Patentansprüche

1. System, umfassend:
eine kabellose medizinische Vorrichtung, die einen Beschleunigungsmesssensor aufweist, der dafür ausgelegt ist, ein epikardiales oder endokardiales Akzelerations(EA)-Signal aus einem Abschnitt eines Herzens zu erfassen, wobei das EA-Signal eine oder mehrere Herztonkomponenten angibt; und
eine Partnervorrichtung, die von der kabellosen medizinischen Vorrichtung getrennt, aber kommunikativ mit dieser verbunden ist, wobei die Partnervorrichtung eine Prozessorschaltung aufweist, die dafür ausgelegt ist, einen Diastolenfunktionsindikator (DFI) unter Verwendung des erfassten EA-Signals zu erzeugen.

2. System nach Anspruch 1, wobei die kabellose medizinische Vorrichtung eine erste Kommunikationsschaltung aufweist, die über eine kabellose Kommunikationsverbindung kommunikativ mit der Partnervorrichtung gekoppelt ist, wobei die erste Kommunikationsschaltung dafür ausgelegt ist, von der Partnervorrichtung einen Erfassungsbefehl zum Erfassen des EA-Signals und zum Senden des erfassten EA-Signals an die Partnervorrichtung zu empfangen.

3. System nach einem der Ansprüche 1-2, wobei der Beschleunigungsmesssensor dafür ausgelegt ist, an einer Oberfläche der kabellosen medizinischen Vorrichtung angebracht zu werden und sich dieser weitgehend anzupassen,
wobei die kabellose medizinische Vorrichtung einen Ankermechanismus zum Positionieren der kabellosen medizinischen Vorrichtung auf einer Endokard- oder Epikardoberfläche eines linken Ventrikels, eines rechten Ventrikels, eines linken Atriums oder eines rechten Atriums aufweist.

4. System nach einem der Ansprüche 1-3, wobei die kabellose medizinische Vorrichtung eine Therapieschaltung aufweist, die dafür ausgelegt ist, als Reaktion auf einen Therapiebefehl von der Partnervorrichtung eine Therapie für den Patienten zu erzeugen.

5. System nach Anspruch 2, wobei der Erfassungsbefehl eine Zeit zum Starten der Erfassung des EA-Signals und eine Zeit zum Beenden der Erfassung des EA-Signals oder von Elektroden, die verwendet werden, um das EA-Signal zu erfassen, einschließt.

6. System nach einem der Ansprüche 1-5, wobei die Partnervorrichtung aufweist:
eine physiologische Sensorschaltung, die dafür ausgelegt ist, ein kardiales Signal vom Herzen zu erfassen;
eine zweite Kommunikationsschaltung, die dafür ausgelegt ist, das erfasste EA-Signal vom Beschleunigungsmesssensor über die drahtlose Kommunikationsverbindung zu empfangen; und
die Prozessorschaltung, die dafür ausgelegt ist, den DFI unter Verwendung des kardialen Signals und des erfassten EA-Signals zu erzeugen.

7. System nach Anspruch 6, wobei:
der physiologische Sensor dafür ausgelegt ist, ein HS-Signal zu erfassen; und
die Prozessorschaltung dafür ausgelegt ist, den DFI unter Verwendung des erfassten EA-Signals und des erfassten HS-Signals zu erzeugen.

8. System nach Anspruch 7, wobei:
die physiologische Sensorschaltung ferner dafür ausgelegt ist, eine kardiale Aktivierung zu erfassen; und die Prozessorschaltung dafür ausgelegt ist, den DFI unter Verwendung von Zeitinformationen über die erfasste kardiale Aktivierung und das erfasste HS-Signal zu erzeugen.

9. System nach Anspruch 8, wobei die Prozessorschaltung ausgelegt ist zum:
Bestimmen eines HS-Erkennungsfensters für einen oder mehrere von einem ersten (S1), einem zweiten (S2), einem dritten (S3) oder einem vierten (S4) Herzton unter Verwendung der Zeiten der kardialen Aktivierungen;
Erkennen des einen oder der mehreren Herztöne in den jeweiligen HS-Erkennungsfenstern und Erzeugen des DFI unter Verwendung des einen oder der mehreren erkannten Herztöne.

10. System nach einem der Ansprüche 1-9, umfassend:
eine erste kabellose medizinische Vorrichtung, die dafür ausgelegt ist, auf einer Endokard- oder Epikardoberfläche eines linken Ventrikels (LV) des Herzens positioniert zu werden, um eine LV-Aktivierung zu erfassen; und
eine zweite kabellose medizinische Vorrichtung, die dafür ausgelegt ist, auf einer Endokard- oder Epikardoberfläche eines rechten Ventrikels (LV) des Herzens positioniert zu werden, um eine RV-Aktivierung zu erfassen,
wobei die Prozessorschaltung der Partnervorrichtung dafür ausgelegt ist, den DFI unter Verwendung einer zeitlichen Beziehung zwischen der erfassten LV-Aktivierung und der erfassten RV-Aktivierung zu erzeugen.

11. System nach einem der Ansprüche 1-9, umfassend:
eine erste kabellose medizinische Vorrichtung, die dafür ausgelegt ist, auf einer Endokard- oder Epikardoberfläche eines Atriums des Herzens positioniert zu werden, wobei die erste kabellose medizinische Vorrichtung einen Beschleunigungsmesssensor aufweist, um ein atriales EA-Signal zu erfassen; und
eine zweite kabellose medizinische Vorrichtung, die dafür ausgelegt ist, auf einer Endokard- oder Epikardoberfläche eines Ventrikels des Herzens positioniert zu werden, wobei die zweite kabellose medizinische Vorrichtung einen Beschleunigungsmesssensor aufweist, um ein ventrikuläres EA-Signal zu erfassen; und
wobei die Prozessorschaltung der Partnervorrichtung dafür ausgelegt ist, den DFI unter Verwendung einer Intensitätsbeziehung zwischen dem erfassten atrialen EA-Signal und dem erfassten ventrikulären EA-Signal zu erzeugen.

12. System nach Anspruch 11, wobei der DFI ein Verhältnis einer Intensität des atrialen EA-Signals zu einer Intensität des ventrikulären EA-Signals einschließt.

13. System nach einem der Ansprüche 1-12, wobei die Partnervorrichtung eine implantierbare kardiale Vorrichtung einschließt.

14. System nach einem der Ansprüche 1-13, wobei die Prozessorschaltung dafür ausgelegt ist, eine sich verschlimmernde Herzschwäche unter Verwendung des DFI zu erkennen.

15. System nach einem der Ansprüche 1-14, wobei die Prozessorschaltung ausgelegt ist zum:
Bestimmen, ob das EA-Signal während einer kardialen Erfassung oder einer kardialen Schrittsteuerung erfasst wird; und
Erzeugen des DFI unter Verwendung von zumindest einem Abschnitt des EA-Signals nur während der kardialen Erfassung.

## Revendications

1. Système, comprenant :
un dispositif médical sans fil qui inclut un capteur d'accéléromètre qui est configuré pour détecter un signal d'accélération épicardiale ou endocardiale (EA) à partir d'une partie d'un cœur, le signal d'EA étant indicatif d'une ou de plusieurs composante(s) de bruit cardiaque ; et
un dispositif compagnon qui est séparé du dispositif médical sans fil et qui lui est couplé en termes de communication, le dispositif compagnon incluant un circuit de processeur qui est configuré pour générer un indicateur de fonction diastolique (DFI) en utilisant le signal d'EA détecté.

2. Système selon la revendication 1, dans lequel le dispositif médical sans fil inclut un premier circuit de communication qui est couplé en termes de communication au dispositif compagnon via une liaison de communication sans fil, le premier circuit de communication étant configuré pour recevoir une commande de détection en provenance du dispositif compagnon pour détecter le signal d'EA et pour transmettre le signal d'EA détecté au dispositif compagnon.

3. Système selon l'une quelconque des revendications 1 et 2, dans lequel le capteur d'accéléromètre est configuré pour être lié à une surface du dispositif médical sans fil et pour se conformer de façon substantielle à cette même surface,
dans lequel le dispositif médical sans fil inclut un mécanisme d'ancrage pour positionner le dispositif médical sans fil sur une surface endocardiale ou épicardiale d'une chambre cardiaque prise parmi un ventricule gauche, un ventricule droit, une oreillette gauche et une oreillette droite.

4. Système selon l'une quelconque des revendications 1 à 3, dans lequel le dispositif médical sans fil inclut un circuit de thérapie qui est configuré pour générer une thérapie pour le patient en réponse à une commande de thérapie en provenance du dispositif compagnon.

5. Système selon la revendication 2, dans lequel la commande de détection inclut un instant pour démarrer la détection du signal d'EA et un instant pour terminer la détection du signal d'EA, ou des électrodes qui sont utilisées pour détecter le signal d'EA.

6. Système selon l'une quelconque des revendications 1 à 5, dans lequel le dispositif compagnon inclut :
un circuit de capteur physiologique qui est configuré pour détecter un signal cardiaque à partir du cœur ;
un second circuit de communication qui est configuré pour recevoir le signal d'EA détecté en provenance du capteur d'accéléromètre via la liaison de communication sans fil ; et
le circuit de processeur qui est configuré pour générer le DFI en utilisant le signal cardiaque et le signal d'EA détecté.

7. Système selon la revendication 6, dans lequel :
le capteur physiologique est configuré pour détecter un signal de HS ; et
le circuit de processeur est configuré pour générer le DFI en utilisant le signal d'EA détecté et le signal de HS détecté.

8. Système selon la revendication 7, dans lequel :
le circuit de capteur physiologique est en outre configuré pour détecter une activation cardiaque ; et le circuit de processeur est configuré pour générer le DFI en utilisant une information de cadencement de l'activation cardiaque détectée et le signal de HS détecté.

9. Système selon la revendication 8, dans lequel le circuit de processeur est configuré pour :
déterminer des fenêtres de détection de HS pour un ou plusieurs bruit(s) cardiaque(s) pris parmi des premier (S1), deuxième (S2), troisième (S3) et quatrième (S4) bruits cardiaques en utilisant les cadencements d'activations cardiaques ;
détecter les un ou plusieurs bruits cardiaques à l'intérieur des fenêtres de détection de HS respectives ; et générer le DFI en utilisant les un ou plusieurs bruits cardiaques détectés.

10. Système selon l'une quelconque des revendications 1 à 9, comprenant :
un premier dispositif médical sans fil qui est configuré pour être positionné sur une surface endocardiale ou épicardiale d'un ventricule gauche (LV) du cœur pour détecter une activation de LV ; et
un second dispositif médical sans fil qui est configuré pour être positionné sur une surface endocardiale ou épicardiale d'un ventricule droit (RV) du cœur pour détecter une activation de RV ;
dans lequel le circuit de processeur du dispositif compagnon est configuré pour générer le DFI en utilisant une relation temporelle entre l'activation de LV détectée et l'activation de RV détectée.

11. Système selon l'une quelconque des revendications 1 à 9, comprenant :
un premier dispositif médical sans fil qui est configuré pour être positionné sur une surface endocardiale ou épicardiale d'une oreillette du cœur, le premier dispositif médical sans fil incluant un capteur d'accéléromètre pour détecter un signal d'EA auriculaire ; et
un second dispositif médical sans fil qui est configuré pour être positionné sur une surface endocardiale ou épicardiale d'un ventricule du cœur, le second dispositif médical sans fil incluant un capteur d'accéléromètre pour détecter un signal d'EA ventriculaire ; et
dans lequel le circuit de processeur du dispositif compagnon est configuré pour générer le DFI en utilisant une relation d'intensités entre le signal d'EA auriculaire détecté et le signal d'EA ventriculaire détecté.

12. Système selon la revendication 11, dans lequel le DFI inclut un rapport d'une intensité du signal d'EA auriculaire sur une intensité du signal d'EA ventriculaire.

13. Système selon l'une quelconque des revendications 1 à 12, dans lequel le dispositif compagnon inclut un dispositif cardiaque implantable.

14. Système selon l'une quelconque des revendications 1 à 13, dans lequel le circuit de processeur est configuré pour détecter une aggravation de l'insuffisance cardiaque en utilisant le DFI.

15. Système selon l'une quelconque des revendications 1 à 14, dans lequel le circuit de processeur est configuré pour :
déterminer si le signal d'EA est détecté pendant une détection cardiaque ou pendant une stimulation cardiaque ; et
générer le DFI en utilisant au moins une partie du signal d'EA seulement pendant la détection cardiaque.
